# EUROPEAN PATENT APPLICATION

(11) **EP 1 992 387 A2**
(43) Date of publication of application: **19.11.2008**
(21) Application number: 08008819.8
(22) Date of filing: 13.05.2008
(51) Int. Cl.: A61M 37/00

(54) **Tattooing machine for acupunctures, tattoos and the like**

(30) Priority: 16.05.2007 IT MI20070990
(71) Applicant: Galvan, Mauro, 20070 Cerro al Lambro (Milano) (IT)
(72) Inventor: Galvan, Mauro, 20070 Cerro al Lambro (Milano) (IT)
(74) Representative: Cicogna, Franco

(57) **Abstract**

A tattooing machine (1) for performing acupunctures, tattoos and the like, comprises a handle body (2) supporting at least a needle (5) and pneumatically driven reciprocating driving means, coupled to at least a needle through an articulated coupling leverage, characterized in that said tattooing machine further comprises adjusting means for adjusting the needle impact frequency and depth, said adjusting means being driven, even with the needle in operation, without contacting the needle.

## Description

### BACKGROUND OF THE INVENTION

The present invention relates to an improved tattooing machine, specifically designed for performing acupunctures, tattoos and the like.

Automatic devices, driven by electric motors, for performing acupunctures and tattoos in general are already known.

The above automatic devices solve some important problems related to a conventional tattooing system, in which the operator manually introduces one or more needles into the skin of a person to be tattooed.

In a conventional manual system, the insertion depth of the needle depends on the skillness of the operator.

In some cases, the tattooing operator performs manually controlled movements which, in addition to being very fatiguing, do not assure an even insertion of the needles into the person skin.

The electrically operated automatic devices, on the other hand, partially solve the above mentioned problems, but are still affected by serious defects.

In fact, the inclusion of an electric motor, prevents the tattooing operator from properly sterilizing said electric motor in an autoclave.

This drawback is a particularly serious one, since, as is known acupunctures, tattoos and the like must be carried out by accurately sterilized apparatus and tools.

Moreover, the provision of an electric motor is such as to increase the weight of the automatic tattooing devices, in addition to complicating a proper handling thereof.

Such a drawback is further aggravated by the provision of power supply electric conductors, which must be properly handled by the tattooing operator.

### SUMMARY OF THE INVENTION

Accordingly, the aim of the present invention is to overcome the above mentioned problems, by providing a tattooing machine for performing acupunctures, tattoos and the like, which has a very small weight and can be easily handled, while allowing to automatically insert the needle or needles into a person skin, by an always constant depth, while allowing to apply an always even and presettable dye amount to the skin of the person being tattooed.

Within the scope of the above mentioned aim, a main object of the invention is to provide such a tattooing machine which allows to greatly simplify the tattooing needle handling operations, in particular allows to automatically introduce the needle or needles into a person's skin, without fatiguing the operator.

Yet another particular object of the present invention is to provide such an improved tattooing machine designed to easily control the impact frequency of the tattooing needle and/or needles on the skin.

Yet another particular object of the present invention is to provide such an improved tattooing machine allowing to easily adjust the operating stroke of the needle or needles penetrating the skin, with the needle or needles in operation, and this without touching or contacting the needle and/or needles.

According to one aspect of the present invention, the above mentioned aim and objects, as well as yet other objects, which will become more apparent hereinafter, are achieved by an improved tattooing machine for acupunctures, tattoos and the like, comprising a handle body supporting at least a needle, pneumatically driven reciprocating driving means, coupled to at least said needle through an articulated coupling leverage, characterized in that said tattooing machine comprises moreover adjusting means for adjusting the needle driving frequency and depth, said adjusting means being driven, even with the needle in operation, without contacting said needle.

### BRIEF DESCRIPTION OF THE DRAWINGS

Further characteristics and advantages of the present invention will become more apparent hereinafter from the following detailed disclosure of a preferred, though not exclusive, embodiment of the invention, which is illustrated, by way of an indicative, but not limitative, example in the accompanying drawings, where:

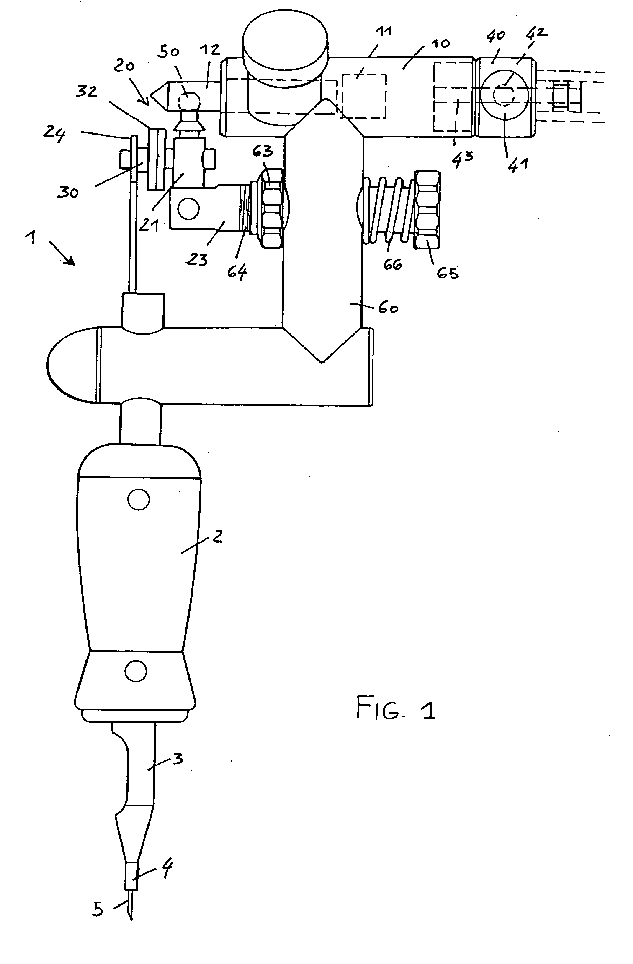

Figure 1 is a side elevation view of the improved tattooing machine according to the present invention;

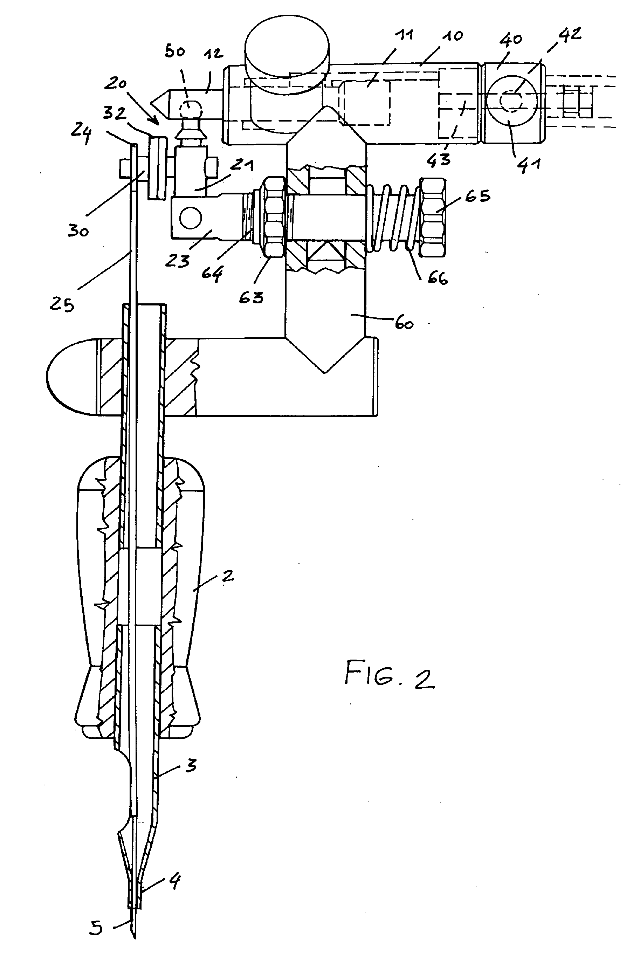

Figure 2 is a further side elevation view, as longitudinally cross-sectioned, of the improved tattooing machine according to the present invention;

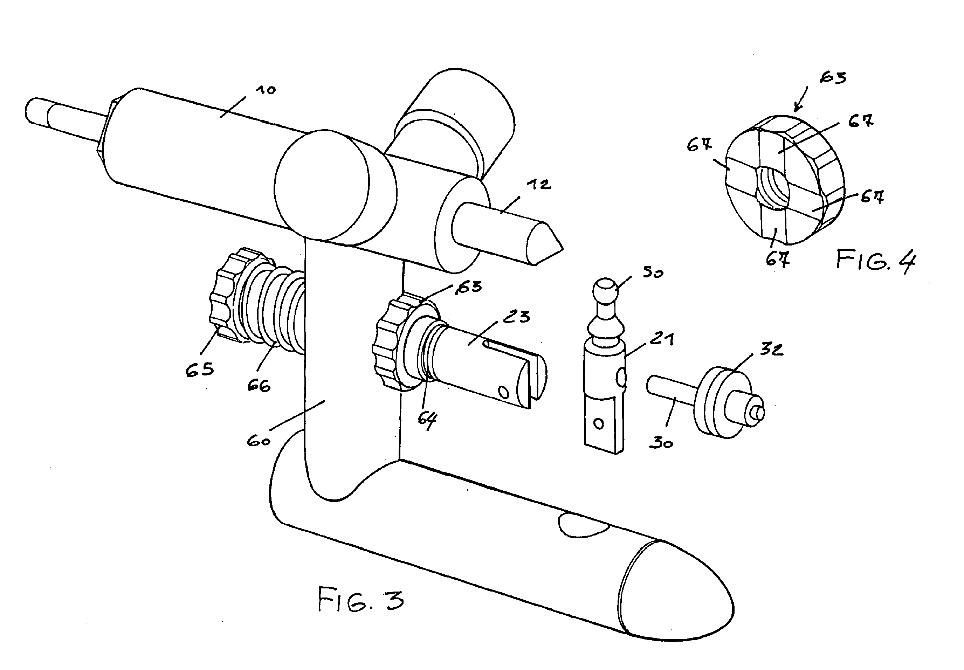

Figure 3 is an exploded perspective view of the improved tattooing machine according to the invention; and
Figure 4 is a further perspective view, on an enlarged scale, showing a detail of a front ring nut element included in the improved tattooing machine according to the present invention.

### DESCRIPTION OF THE PREFERRED EMBODIMENTS

With reference to the number references of the above mentioned figures, the improved tattooing machine, according to the present invention, which has been generally indicated by the reference number 1, comprises a handle body 2 of elongated contour, including, at an end portion thereof, a guide tube element 3, ending with a channel 4, therethrough a needle 5 can be projected.

In particular, said needle can also comprise a plurality of micro needles, arranged in an adjoining mutual relationship, thereby providing a so-called needle beam.

To the handle body 2 driving means, in particular pressurized air driven reciprocating driving means, are coupled.

Said driving means comprise, in particular, fixedly connected to said handle, a driving plunger 11, the stem 12 of which is coupled to an articulated leverage assembly, generally indicated by the reference number 20.

Said articulated leverage assembly 20 comprises a connecting crank rod 11, pivoted at an end portion of the plunger stem or rod 12 and further connected, at the other end portion thereof, to an arm element 23 associated with an upright 60 of the handle body.

Said arm element 23 can be driven along an axis thereof and cannot turn with respect to the upright 20 of the handle body.

To that end, the arm element 23 comprises an axial slot 61 engaged by a pin 62 axially arranged in the upright 60 and suitable to prevent said arm element from turning about its axis.

As shown, the arm element 23 is provided at a front thereof with a front ring-nut 63, engaging with a thread 64 of said arm element 23.

At a rear portion thereof, the arm element 23 comprises a rear ring nut 65 defining an engagement flange for engaging a spring 60 coaxially extending with respect to said arm element.

In particular, said spring 66 operates between the upright 60 and the rear or back ring-nut 65, so as to urge said arm element 23 to a rear use position.

Thus, by turning the front ring nut 63, the arm element 23 is displaced or driven, thereby changing its position with respect to the upright 60, thereby in turn changing the geometrical configuration of said articulated leverage assembly 20.

Advantageously, the front ring nut 63 comprises a plurality of contoured slots 67, arranged at 90° with respect to one another, so as to allow the front ring nut 63 to abut against the upright 60, which has a cylindric configuration, to cause the front ring nut 63 to assume a plurality of stable positions at a 1/4 of revolution.

As shown, the connecting rod 21 is pivoted to an end portion of the stem or plunger rod 12 by a spherical joint including a ball element 50 rigid with the connecting rod 21 and engaging in a seat formed in the body of the plunger stem or rod 12.

Advantageously, said spherical joint is made of an anti-wear material such as Teflon or a thermally resistant plastics material.

Thus, said spherical joint allows to reduce the number of components forming the subject device, while allowing to assemble in a very quick and simple manner said device.

Moreover, said spherical joint will greatly reduce the noise of the device or instrument as it is used, while greatly increasing the instrument lifetime, since it is a highly mechanically stressed instrument or device.

An adjustable lever arm 30 connects the crank connecting rod 21 to a guide rod 25, through an eye arrangement 24, said rod 25 supporting said needle and/or needles 5.

Thus, as the plunger 11 is reciprocatedly driven, the articulated leverage assembly 20 will in turn reciprocatedly drive the needle or needle assembly 5, thereby causing the needle or needle assembly to be urged to a preset depth into the skin of the person being tattooed.

In particular, this penetration depth can be properly adjusted by operating the adjustable lever arm 30, by merely turning a flange 32.

The reciprocating movement frequency can be herein also adjusted by operating a frequency adjusting device 40 applied to the pneumatic cylinder 10.

Said adjusting device 40, in particular, comprises a cock element 41, affecting a stem 42 on the pressurized air passage 43, so as to change the cross-section of the latter.

Thus, the adjusting system of the arm element 23 allows to easily and quickly mount a disposable needle since, by pushing and then releasing the back or rear ring nut 65, the support 30 and eye assembly 24 can be easily mutually engaged.

Moreover, the arm element 23 adjusting system allows to easily change, even with the needle in an assembled condition, the operating needle depth, that is the needle skin penetration, even during the tattooing operating step, that is with an operating needle.

This is very important to optimize tattooed lines and shades.

A further advantage of the adjustable arm element 23 is that the adjustment is performed in a very simple and quick manner, while limiting a possible viral pollution of the tattooing instrument handle.

Yet another important advantage of the arm element 23 is that the impact of the needle onto the skin is not a rigid one, but is cushioned by a cushioning spring 66.

It has been found that the invention fully achieves the intended aim and objects.

In fact, the invention provides a tattooing machine, driven by driving means including a very simple pneumatic cylinder, which can provide a reciprocating movement by pressurized air.

Thus, said driving means are very reliable in operation.

Moreover, the tattooing machine according to the present invention can be quickly and easily sterilized also in an autoclave and, moreover, is very competitive from a mere economic standpoint.

In particular, the fact is to be pointed out that the tattooing machine according to the present invention, in addition to being very simple and light, is very efficient since it allows the needle or needles to easily and quickly penetrate the skin, with an insertion depth which can be changed at will, owing to the provision of the needle penetration adjusting means.

In particular, said adjusting means will provide perfectly patterned tattoos, that is tattoos with perfectly defined pattern lines, features and shades.

The above advantageous characteristics, which are very important for performing good tattoos, are merely achieved by changing the impact frequency of the needle or needles on the skin, while properly changing the needle penetration depth.

Furthermore, the cock device 40 allows to simply and quickly change the needle impact frequency; moreover the cock device 40 is an integrating part of the tattooing instrument, it can be sterilized with the latter.

To the above it is to be further added that the adjustable lever 30 can be easily operated to properly change the needle penetration depth, and can also be sterilized together with the overall tattooing instrument.

In practicing the invention, the used materials, as well as the contingent size and shapes, can be any, depending on requirements.

## Claims

1. An improved tattooing machine for acupunctures, tattoos and the like, comprising a handle body supporting at least a needle, and pneumatically driven reciprocating driving means, coupled to said at least a needle through an articulated coupling leverage, **characterized in that** said tattooing machine comprises moreover adjusting means for adjusting the needle impact frequency and depth, said adjusting means being adapted to be driven, with the needle operation, without contacting said needle.

2. A tattooing machine, according to claim 1, **characterized in that** said driving means comprise a pneumatic cylinder fixedly connected to a handle and driving a plunger having a plunger rod coupled to an articulated leverage assembly.

3. A tattooing machine, according to claim 2, **characterized in that** said articulated leverage assembly comprises a crank piston rod pivoted at an end portion thereof to said plunger rod and further connected, at the other end portion thereof, to an arm element associated with an upright of said handle body.

4. A tattooing machine, according to one or more of the preceding claims, **characterized in that** said needle impact frequency and depth adjusting means comprise an adjustable lever arm connecting said crank rod to a guide rod through an eye assembly, and that said guide rod supports said at least a needle.

5. A tattooing machine, according to one or more of the preceding claims, **characterized in that** said adjustable lever arm comprises a nut screw body having a nut screw nut body flange and designed for engaging a threaded body thereby, by turning said flange, a length of said lever arm is changed.

6. A tattooing machine, according to one or more of the preceding claims, **characterized in that** said plunger is so reciprocatedly driven that said articulated leverage assembly in turn reciprocately drives said needle, to cause said needle to enter said skin for a preset depth, said depth being adjustable by said adjustable lever arm and flange.

7. A tattooing machine, according to one or more of the preceding claims, **characterized in that** said needle impact frequency and depth adjusting means comprise an adjusting device, coupled to said pneumatic cylinder and including a cock element cooperating with a stem in a pressurized air passage, to change a cross section of said pressurized air passage.

8. A tattooing machine, according to one or more of the preceding claims, **characterized in that** said at least a needle is slidably housed in said handle body and ends with a tubular portion defining a guide channel for said at least a needle.

9. A tattooing machine, according to one or more of the preceding claims, **characterized in that** said at least needle comprises a plurality of microneedles.

10. A tattooing machine, according to one or more of the preceding claims, **characterized in that** said arm element, coupled to said handle body upright, is movable along an axis thereof without turning with respect to said upright.

11. A tattooing machine, according to one or more of the preceding claims, **characterized in that** said arm element comprises an axial slot engaged by an engagement pin axially arranged on said upright and preventing said arm element from turning about its axis.

12. A tattooing machine, according to one or more of the preceding claims, **characterized in that** said arm element comprises, at a front portion thereof, a front ring nut engaging a thread of said arm element and including, at a rear portion thereof, a rear ring nut providing a flange for engaging a spring coaxial with said arm element, said spring operating between said upright and rear ring nut to urge said arm element to a backward use position, said front ring nut, as it is rotatively driven, displacing said arm element so as to change a position of said arm element with respect to said upright to in turn change a geometry of said articulated leverage assembly.

13. A tattooing machine, according to one or more of the preceding claims, **characterized in that** said front ring nut comprises a plurality of contoured slots arranged at 90° with respect to one another, thereby allowing said front ring nut to abut on said upright, said upright having cylindric configuration, thereby assuming a plurality of stable positions at 1/4 of a revolution.

14. A tattooing machine, according to one or more of the preceding claims, **characterized in that** said crank rod is pivoted to one portion of said plunger rod by a spherical joint including a ball element rigid with said crank rod and engaging in a seat formed in said plunger rod.

15. A tattooing machine, according to one or more of the preceding claims, **characterized in that** said spherical joint is made of an antiwear material, such Teflon or other thermally resistant plastics material.
